# EUROPÄISCHE PATENTSCHRIFT

(11) **EP 0 830 838 B1**
(45) Veröffentlichungstag und Bekanntmachung des Hinweises auf die Patenterteilung: **22.10.2003**
(21) Anmeldenummer: 97112331.0
(22) Anmeldetag: 18.07.1997
(51) Int. Cl.: A61B 3/06, A61B 3/032

(54) **Sehtestgerät**
Vision test apparatus
Appareil d'essai de la vision

(30) Priorität: 20.09.1996 DE 29616443 U
(43) Veröffentlichungstag der Anmeldung: 25.03.1998
(73) Patentinhaber: Oculus Optikgeräte GmbH, 35582 Wetzlar (DE)
(72) Erfinder: Kirchhübel, Rainer, Dipl.-Ing., 35614 Asslar (DE); Feiertag, Carsten, Dipl.-Ing., 35410 Hungen (DE)
(74) Vertreter: Missling, Arne, Dipl.-Ing.

(56) Entgegenhaltungen:
- US-A- 5 485 231

## Beschreibung

Die Erfindung betrifft ein Sehtestgerät, insbesondere zur Prüfung des Dämmerungssehens mit einem in den Strahlengang einzuschaltenden Sehzeichen, mit einer neben dem Sehzeichen angeordneten wahlweisen zuund wegschaltbaren Blendlichtquelle, mit einem geschlossenen Gehäuse, das mit einer Einblicköffnung für die Testperson und einer in Verlängerung der Augenachse liegenden Öffnung versehen ist, die zwar ein ungehindertes Hinaussehen gestattet, jedoch mit einem Filter zur Abschwächung des von außen einfallenden Lichtes versehen ist, wobei in Verlängerung der Augenachse ein hierzu geneigter teildurchlässiger Spiegel angeordnet ist und in den vom Auge zum Sehzeichen führenden Strahlengang ein lichtbrechendes Element angeordnet ist.

Ein Sehtestgerät der eingangs genannten Art, das zur Prüfung der Dämmerungssehschärfe und Blendempfindlichkeit dient, ist aus der DE-C-30 03 588 bekannt. Bei diesem Sehtestgerät dient zur Prüfung des Dämmerungssehens und der Blendempfindlichkeit ein Schirm, der von zwei Projektoren gleichzeitig beleuchtet wird, wobei im Strahlengang des einen Projektors ein Sehzeichen angeordnet ist und die Lichtstärken beider Projektoren so miteinander gekoppelt sind, daß die Lichtstärke des einen Projektors in der gleichen Weise zunimmt, wie die des anderen Projektors abnimmt. Dieses bekannte Sehtestgerät, das ein Freisichtgerät ist, hat einen relativ hohen baulichen Aufwand, erfordert vier Projektoren und viele Umlenkspiegel zur Erzeugung der gewünschten Sehzeichen. Dieser relativ große bauliche Aufwand bedingt auch ein großes Gehäuse und eine relativ schwere Konstruktion, so daß dieses auch nur bedingt transportierbar ist.

Des weiteren sind aus der DE-A 24 09 747 und der DE-A 2 321 570 Sehtestgeräte bekannt, die ein geschlossenes Gehäuse aufweisen, jedoch nicht das Freisehen gewährleisten. Darüber hinaus erfordern diese Geräte, daß vor jedem Auge ein Linsenpaar angeordnet ist, welches sehr genau fokussiert werden muß, damit das Sehzeichen als Einheit gesehen wird und nicht ein Sprung oder ein Versatz im Sehzeichen auftritt. Derartige Geräte sind zum einen aufwendig zu justieren und darüber hinaus auch stoßempfindlich, so daß diese nur beschränkt transportabel sind. Bekannt ist es aus diesen Druckschriften, die Sehzeichen auf einem scheibenförmigen Träger anzuordnen, der von einer Lichtquelle durchleuchtet wird.

Der Erfindung liegt die Aufgabe zugrunde, ein Sehtestgerät der eingangs genannten Art so auszubilden, daß dieses zum einen kompakt und leicht und des weiteren robust im Aufbau ist, das es erlaubt, die Dämmerungssehschärfe und die Blendempfindlichkeit zu testen und zusätzlich als Variante die Tagessehschärfe oder abgestufte Visuswerte unter Dämmerungsbedingungen zu ermitteln. Eine weitere Aufgabe der Erfindung besteht darin, das Sehtestgerät so auszubilden, daß es rechnergesteuert betrieben und somit der Prüfungsaufwand erheblich reduziert werden kann.

Diese Aufgabe wird erfindungsgemäß durch die Merkmale des kennzeichnenden Teils des Anspruchs 1 gelöst.

Gemäß der Erfindung werden die Sehzeichen auf einem Testträger aufgebracht, vorteilhaft aufgedampft, wobei diese Sehzeichen durchleuchtet werden und zwar von einer einzigen Lichtquelle, wobei ein Beleuchtungsstrahlengang erhalten wird, in dem vorteilhaft eine einzige achromatische Linse angeordnet ist. In dem Strahlengang ist des weiteren ein teildurchlässiger Spiegel angeordnet, der in Verlängerung der Augenachse liegt, so daß ein Freisichtgerät durch eine in Augenachse des Gerätes liegende Öffnung erhalten wird. Diese Öffnung ist durch eine Grauscheibe verschlossen, so daß das Tageslicht nur gedämpft eintreten kann. Die Einblicksöffnung ist gleichfalls durch eine durchsichtige Scheibe verschlossen, so daß das ganze Gerät hermetisch abgeschlossen und somit gegen Staubeintritt geschützt ist. Hierdurch entfallen aufwendige Wartungsarbeiten, wie sie beim Stand der Technik notwendig sind. Dies wird des weiteren dadurch erreicht, daß die Einstellung der Sehzeichen sowie der Filter wie auch der Lochblenden motorisch erfolgt, so daß das Gehäuse keine weiteren Öffnungen für Handhaben etc. aufweisen muß. Alle möglichen Testvarianten sind auf dem Träger für die Sehzeichen untergebracht, so daß diese z. B. per Schrittmotor und Tastatur oder alternativ durch einen Computer ansteuerbar sind. Diese Ansteuerung hat auch gleichzeitig den Vorteil, daß die kompletten Daten als fertiges Ergebnis an eine Datenverarbeitungseinheit übertragen werden kann, so daß der Aufwand für die Bedienungsperson minimiert wird.

Das Gerät kann gleichfalls Testzeichen zur Tagessehschärfe enthalten, so daß das Gerät als Kombinationsgerät eingesetzt werden kann, so daß hier die Tagessehschärfe wie auch die Dämmerungssehschärfe und die Blendempfindlichkeit geprüft werden können. Weitere vorteilhafte Ausgestaltungen der Erfindung gehen aus den Unteransprüchen in Verbindung mit den Zeichnungen und der Beschreibung hervor.

Ein Ausführungsbeispiel der Erfindung ist im folgenden anhand der Zeichnung näher beschrieben. In dieser zeigen
- Fig. 1: eine Seitenansicht im Schnitt eines erfindungsgemäßen ausgebildeten Gerätes,
- Fig. 2: eine Seitenansicht des unteren Teiles des Gerätes nach Fig. 1, unter Weglassung der Gehäusekontur,
- Fig. 3: einen Schnitt nach Linie III-III in Fig. 2 und
- Fig. 4: ein weiteres Ausführungsbeispiel gemäß der Erfindung.

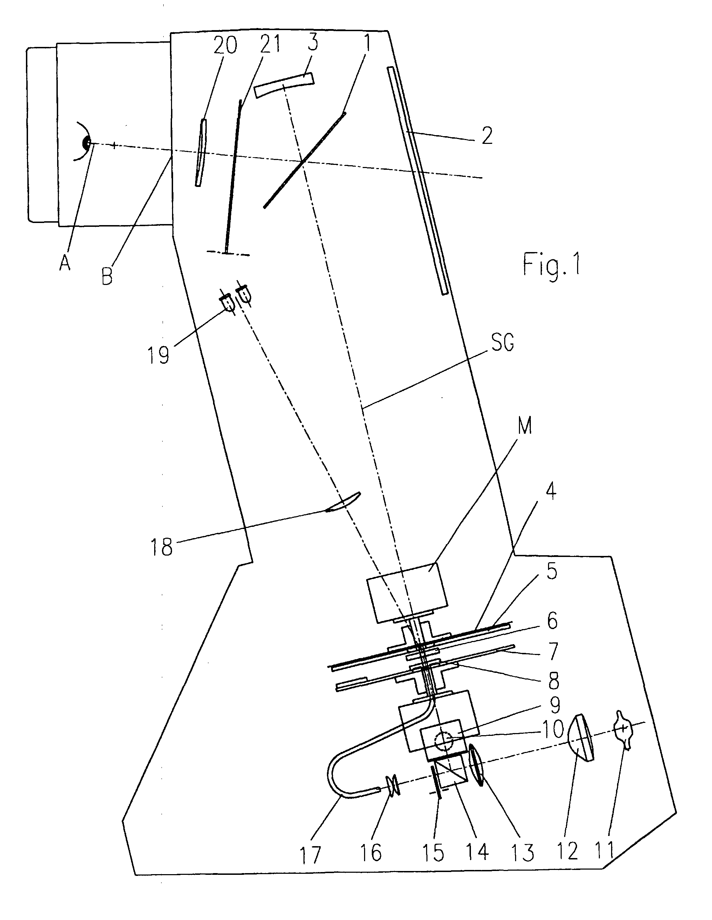

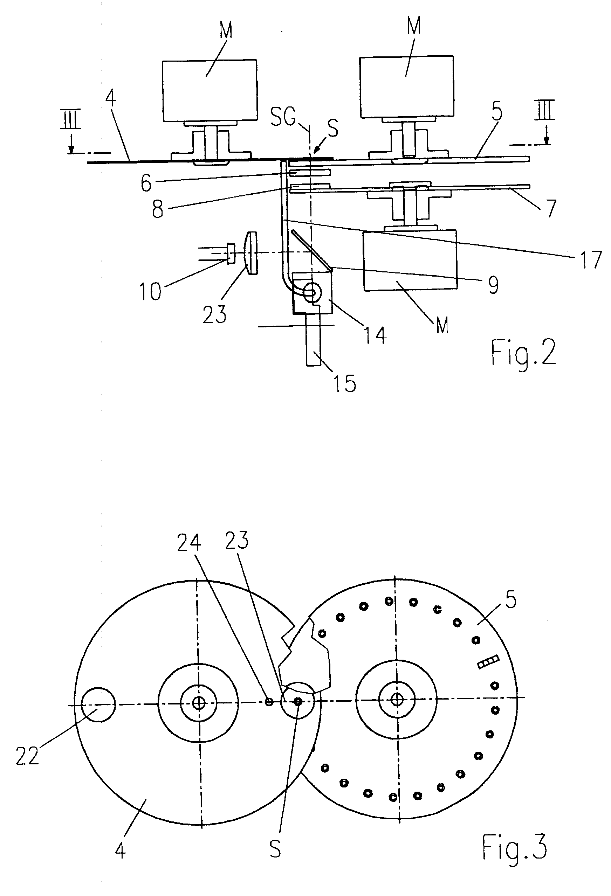

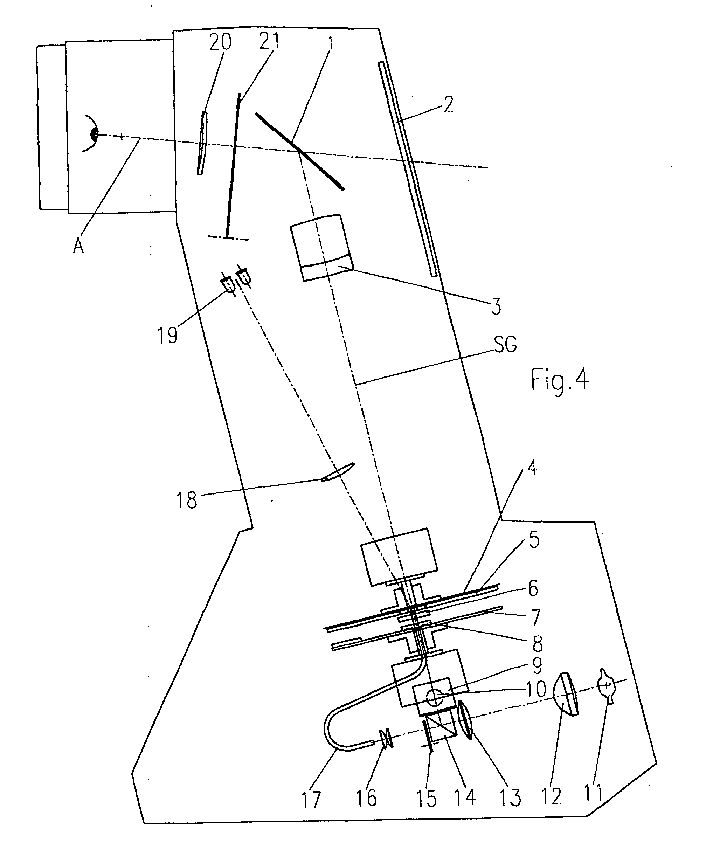

In der Fig. 1 ist eine Seitenansicht eines erfindungsgemäßen Sehtestgerätes im Schnitt dargestellt, wobei im Gerät eine Durchblicksöffnung B für das Auge vorgesehen ist. In Verlängerung der Augenachse A ist ein teildurchlässiger Spiegel 1 angeordnet. Des weiteren weist das Gerät in Verlängerung der Augenachse eine Austrittsöffnung auf, in die eine Grauscheibe eingesetzt ist. Diese Grauscheibe dient dazu, das einfallende Tageslicht zu reduzieren, so daß dieses Gerät in beliebig beleuchteten Räumen einsetzbar ist. In dem vom Auge zum Sehzeichen S (siehe Fig. 2 und 3) führenden Strahlengang SG ist in Fig. 1 ein Hohlspiegel 3 eingesetzt und im Ausführungsbeispiel nach Fig. 4 eine achromatische Linse 3, wobei das Sehzeichen S im Brechpunkt des Hohlspiegels bzw. des Achromaten angeordnet ist. Die achromatische Linse wie auch der Hohlspiegel erstreckt sich über die gesamte Gesichtsfeldbreite. Abgesehen davon, daß das Ausführungsbeispiel nach Fig. 1 einen Hohlspiegel aufweist und das Ausführungsbeispiel nach Fig. 4 eine achromatische Linse, sind die Ausführungsbeispiele ansonsten gleich aufgebaut. Die Fig. 3 und 4 beziehen sich somit gleichermaßen auf das Ausführungsbeispiel nach Fig. 1, wie auch auf das nach Fig. 4.

Die Sehzeichen S sind auf einem Sehzeichenträger 5 angeordnet, der als Scheibe ausgebildet ist, wobei dieser Sehzeichenträger 5 von einem Motor M angetrieben wird. Der Motor M ist ein Schrittmotor, der die Scheibe um vorgegebene Winkelstellungen verdreht, so daß das Sehzeichen im Strahlengang SG zu liegen kommt. Auf einer zweiten Blendenscheibe 4 sind zwei Öffnungen 22 und 23 gleicher Größe für die Sehzeichen S vorgesehen. Der Unterschied der beiden Blendenöffnungen besteht nur darin, daß neben der Blendenöffnung 23 eine weitere Blendenöffnung 24 ausgebildet ist, die das Ende des Lichtleiters 17 freigibt, der die Blendlichtquelle bildet. Je nach Stellung der Blendenscheibe 4 ist entweder die Lichtquelle 11 verdunkelt oder aber zusammen mit der oder ohne Blendlichtquelle 17 freigegeben. Die Blendenscheibe 4 wird gleichermaßen von einem Schrittmotor angetrieben.

Im Strahlengang SG und unterhalb des Sehzeichens S ist eine Mattscheibe 6 angeordnet, die zur Vergleichmäßigung des aus der Lichtquelle 11 austretenden Lichtes dient. Koaxial mit der Sehtestscheibe 5 ist eine Filterscheibe 7 angeordnet, die einen Graufilter 8 trägt. Diese Filterscheibe 7 wird gleichfalls durch einen Schrittmotor M angetrieben, wobei dieser den Graufilter 8 entweder in den Strahlengang SG einschaltet oder aus diesem entfernt. Durch diesen Graufilter 8 wird ein anderes Umfeld auf dem Testfeld erzeugt.

Als Lichtquelle dient eine zentrierte Glühlampe 11, die über einen Kondensor 12 und eine weitere Linse 13 und einen Teilerwürfel 14 auf dem das Sehzeichen tragende Testfeld abgebildet wird. Über den Teilerwürfel 14 wird ein gewisser Lichtanteil ausgeblendet und über einen Kondensor 16 einem Lichtleiter 17 zugeführt, dessen anderes Ende als Blendlichtquelle dient. Vor dem Lichtleiter 17 ist ein Kondensor 16 angeordnet. Des weiteren liegt vor dem Kondensor eine Blende 15, die mehr oder weniger in den Lichtstrahl eingeschwenkt werden kann, um die Helligkeit einzustellen. Aus der Fig. 2 ist ersichtlich, daß zwischen dem Teilerwürfel 14 und dem Sehzeichen S ein teildurchlässiger Spiegel 9 angeordnet ist. Über diesem teildurchlässigen Spiegel wird ein Teil des Lichtstromes ausgeblendet und über eine Linse 23 einer Fotodiode 10 zugeführt. Diese Fotodiode erfaßt die Lichtstärke, so daß diese auf ihren Sollwert eingeregelt werden kann.

Oberhalb der Sehtestscheibe 5 sind zwei Leuchtdioden 19 angeordnet, die über eine Linse 18 neben dem Sehzeichen S abgebildet werden. Diese Leuchtdioden dienen zur Demonstration der Blickrichtung, da in der Regel das Testfeld sehr dunkel ist.

Zur Untersuchung der Dämmerungsmyopie können zusätzlich in den Strahlengang vor den Augen zwei Linsenpaare 20 vorgeschaltet werden, um festzustellen, ob der Seheindruck für den Probanden dadurch besser wird. Dieser Test wird vorzugsweise ohne Blendung durchgeführt. Zusätzlich können hier weitere Blenden 21 in den Strahlengang eingeschwenkt werden, so daß der Test auch monokular durchgeführt werden kann, um vorzugsweise auch Intraokularlinsen-Patienten zu prüfen.

Alle Antriebe sind motorgesteuert und können von einem Bedienfeld aus per Tastendruck angewählt werden. In bestimmter Reihenfolge kann der Test auch vorgegeben ablaufen. Alternativ kann an Stelle eines Ansteuergerätes auch direkt ein Computer eingesetzt werden, wobei der Computer dann die Motoren M direkt ansteuert, wobei alles das, was der Proband auf dem Test erkennen kann, auch für den Untersucher außen auf dem Bildschirm ersichtlich ist. Hierdurch ist eine direkte bessere Kommunikation möglich, wobei die Testergebnisse direkt abgespeichert werden können. Verwechslungen durch falsches Ankreuzen oder Eintragen des Ergebnisses von Blendung bei nicht untersuchter Blendung sind somit absolut ausgeschlossen.

## Patentansprüche

1. Sehtestgerät, insbesondere zur Prüfung des Dämmerungssehens mit einem in den Strahlengang (SG) einzuschaltenden Sehzeichen (S), mit einer neben dem Sehzeichen (S) angeordneten wahlweisen zu- und wegschaltbaren Blendlichtquelle (17), mit einem geschlossenen Gehäuse, das mit einer Einblicköffnung (B) für die Testperson und einer in Verlängerung der Augenachse (A) liegenden Öffnung versehen ist, die zwar ein ungehindertes Hinaussehen gestattet, jedoch mit einem Filter (2) zur Abschwächung des von außen einfallenden Lichtes versehen ist, wobei in Verlängerung der Augenachse (A) ein hierzu geneigter teildurchlässiger Spiegel (1) angeordnet ist und in den vom Auge zum Sehzeichen führenden Strahlengang (SG) ein lichtbrechendes Element (3) angeordnet ist, **dadurch gekennzeichnet, daß**
das Sehtestgerät mehrere beliebig in den Strahlengang (SG) einzuschaltende Sehzeichen (S) umfaßt, daß die Sehzeichen (S) auf einen Träger (5) aufgebracht und von einer Lichtquelle (11) durchleuchtet sind, daß der Träger (5) für die Sichtzeichen derart verschwenk- oder verschiebbar angeordnet ist, daß beliebige auf den Träger (5) aufgebrachte Sehzeichen (S) in den Strahlengang (SG) bringbar sind, daß
das lichtbrechende Element (3) eine in den Strahlengang eingeschaltete, den Sichtbereich beider Augen überdeckende, achromatische Linse oder ein Hohlspiegel ist, in dessen Brennpunkt sich das sich im Strahlengang befindende Sehzeichen (s) befindet, daß in den Strahlengang zwischen Lichtquelle (11) und Sehzeichen (S) ein Filter (8), insbesondere ein Graufilter einschwenkbar ist, daß die Blendlichtquelle (17) stationär neben dem durchleuchteten sich im Strahlengang befindenden Sehzeichen (S) angeordnet ist und daß oberhalb des Trägers (5) eine oder mehrere Blenden (4) mit unterschiedlichen Blendenöffnungen vorgesehen sind, die wahlweise in den Strahlengang (SG) zwischen Auge und schzeichen (s) schiebbar sind.

2. Sehtestgerät nach Anspruch 1, **dadurch gekennzeichnet, daß** die Blendlichtquelle (17) und das Sehzeichen (S) von der gleichen Lichtquelle (11) beleuchtet sind.

3. Sehtestgerät nach Anspruch 2, **dadurch gekennzeichnet, daß** die Blendlichtquelle (17) einen Lichtleiter aufweist, der über einen Teilerspiegel oder einen Teilerwürfel (14) mit der Lichtquelle (11) gekoppelt ist.

4. Sehtestgerät nach einem der Ansprüche 2 oder 3, **dadurch gekennzeichnet, daß** die Lichtstärke der Blendlichtquelle (17) über eine in den Strahlengang zum Lichtleiter einschiebbare Blende (15) einstellbar ist.

5. Sehtestgerät nach einem der Ansprüche 1 bis 4, **dadurch gekennzeichnet, daß** die Helligkeit der Lichtquelle (11) von einem Meßgerät (10) erfaßt und in Abhängigkeit eines vorgegebenen Sollwertes regelbar ist.

6. Sehtestgerät nach einem der Ansprüche 1 bis 5, **dadurch gekennzeichnet, daß** in den Strahlengang (SG) und unter dem Sehzeichen (S) eine Mattscheibe eingeschaltet ist.

7. Sehtestgerät nach einem der Ansprüche 1 bis 6, **dadurch gekennzeichnet, daß** der Träger (5) der Sehzeichen (S) eine Scheibe ist, auf deren Umfang verteilt die Sehzeichen aufgebracht sind.

8. Sehtestgerät nach Anspruch 7, **dadurch gekennzeichnet, daß** die Scheibe (5) aus einem transparenten Material besteht.

9. Sehtestgerät nach einem der Ansprüche 1 bis 8, **dadurch gekennzeichnet, daß** die Blende (4) in Form einer mit Durchbrüchen versehenen Scheibe ausgebildet ist.

10. Sehtestgerät nach den Ansprüchen 7 und 9 oder den Ansprüchen 8 und 9, **dadurch gekennzeichnet, daß** die Blendenscheibe (4) und die Sehtestträgerscheibe (5) achsparallel und entweder koaxial oder aber mit einem Abstand zueinander angeordnet sind.

11. Sehtestgerät nach einem der Ansprüche 7 bis 10, **dadurch gekennzeichnet, daß** der Filter (8) auf einer weiteren Scheibe (7) angeordnet ist, die zumindest koaxial zu der Blendenscheibe (4) oder Sehzeichenträgerscheibe (5) angeordnet ist.

12. Sehtestgerät nach einem der Ansprüche 7 bis 11, **dadurch gekennzeichnet, daß** alle Scheiben (4,5,7) motorisch (M) angetrieben sind.

13. Sehtestgerät nach einem der Ansprüche 7 bis 12, **dadurch gekennzeichnet, daß** die Steuerung des Antriebs der Scheibe oder der Antriebe (M) der Scheiben rechnergesteuert erfolgt.

14. Sehtestgerät nach einem der Ansprüche 1 bis 13, **dadurch gekennzeichnet, daß** oberhalb der Sehzeichen ein oder mehrere Lichtquellen (19) vorgesehen sind, die über Abbildungssysteme (18) Fixationspunkte neben den Sehzeichen (S) bilden.

15. Sehtestgerät nach Anspruch 14, **dadurch gekennzeichnet, daß** die Lichtquellen (19) Dioden sind.

16. Sehtestgerät nach einem der Ansprüche 1 bis 15, **dadurch gekennzeichnet, daß** in dem Strahlengang (SG) zwischen Auge und Sehzeichen (S) ein oder mehrere Blenden (21) zur Abdeckung eines Auges einschiebbar sind.

17. Sehtestgerät nach einem der Ansprüche 1 bis 16, **dadurch gekennzeichnet, daß** in Strahlengang (SG) vor den Augen Meniskenlinsenpaare (20) einschwenkbar angeordnet sind.

## Claims

1. A sight test device, in particular for testing mesopic vision, comprising an optotype (S) to be inserted into the beam path (SG), a glare source (17) arranged next to the optotype (S) and switchable on and off as required, a closed housing provided with a viewing opening (B) for the test person and with an opening which lies on an extension of the optical axis (A) and permits an unimpeded view outside, but which is provided with a filter (2) for reducing the light coming in from outside, wherein a semi-reflecting mirror (1) is arranged on an extension of the optical axis (A) at an angle thereto and a refractive element (3) is arranged in the beam path (SG) leading from the eye to the optotype, **characterised in that** the sight test device comprises a plurality of optotypes (S) to be inserted randomly into the beam path (SG), **in that** the optotypes (S) are applied to a carrier (5) and are transilluminated by a light source (11), **in that** the carrier (5) for the optotypes is pivotably or displaceably arranged so that random optotypes (S) applied to the carrier (5) are movable into the beam path (SG), **in that** the refractive element (3) is an achromatic lens or concave mirror which is inserted into the beam path and covers the visual range of both eyes and in the focal point of which is positioned the optotype (S) located in the beam path, **in that** a filter (8), in particular a grey filter, is pivotable into the beam path between the light source (11) and the optotype (S), **in that** the glare source (17) is fixedly arranged next to the transilluminated optotype (S) located in the beam path and **in that** one or more diaphragms (4) with different diaphragm apertures are provided above the carrier (5) and are insertable into the beam path (SG) between the eye and the optotype (S) as required.

2. A sight test device according to claim 1, **characterised in that** the glare source (17) and the optotype (S) are illuminated by the same light source (11).

3. A sight test device according to claim 2, **characterised in that** the glare source (17) has a light guide connected to the light source (11) via a beam-splitting mirror or a beam-splitting cube (14).

4. A sight test device according to either one of claims 2 and 3, **characterised in that** the light intensity of the glare source (17) is adjustable by means of a diaphragm (15) which is insertable into the beam path leading to the light guide.

5. A sight test device according to any one of claims 1 to 4, **characterised in that** the brightness of the light source (11) is determined by a measuring instrument (10) and is adjustable as a function of a predetermined desired value.

6. A sight test device according to any one of claims 1 to 5, **characterised in that** a ground glass plate is inserted into the beam path (SG) below the optotype (S).

7. A sight test device according to any one of claims 1 to 6, **characterised in that** the carrier (5) for the optotypes (S) is a plate, around the circumference of which are distributed the optotypes.

8. A sight test device according to claim 7, **characterised in that** the plate (5) consists of a transparent material.

9. A sight test device according to any one of claims 1 to 8, **characterised in that** the diaphragm (4) is in the form of a plate provided with apertures.

10. A sight test device according to claims 7 and 9 or claims 8 and 9, **characterised in that** the diaphragm plate (4) and the sight-test carrier plate (5) are arranged so as to be axially parallel and are either co-axial with one another or are spaced apart.

11. A sight test device according to any one of claims 7 to 10, **characterised in that** the filter (8) is arranged on a further plate (7) arranged co-axially at least with the diaphragm plate (4) or the optotype carrier plate (5).

12. A sight test device according to any one of claims 7 to 11, **characterised in that** all the plates (4, 5, 7) are driven by motors (M).

13. A sight test device according to any one of claims 7 to 12, **characterised in that** the drive of the plate or the drives (M) of the plates are computer-controlled.

14. A sight test device according to any one of claims 1 to 13, **characterised in that** one or more light sources (19) are provided above the optotypes and form fixation points next to the optotypes (S) via imaging systems (18).

15. A sight test device according to claim 14, **characterised in that** the light sources (19) are diodes.

16. A sight test device according to any one of claims 1 to 15, **characterised in that** one or more diaphragms (21) for covering an eye are insertable into the beam path (SG) between the eye and the optotype (S).

17. A sight test device according to any one of claims 1 to 16, **characterised in that** meniscus lens pairs (20) are arranged so as to be pivotable into the beam path (SG) in front of the eyes.

## Revendications

1. Appareil de test de vision, en particulier pour le contrôle de la vision mésopique comportant un optotype (S) à interposer dans le trajet de rayons (SG), une source de lumière crue (17), apte à être au choix connectée ou déconnectée et disposée à côté de l'optotype (S), un boîtier fermé qui est doté d'un orifice monoculaire (B) pour l'individu testé et d'un orifice situé dans le prolongement de la ligne de collimation (A), qui permet certes une vision extérieure libre, mais est cependant équipé d'un filtre (2) pour l'atténuation de la lumière incidente de l'extérieur, un miroir partiellement transparent (1) étant incliné dans le prolongement de la ligne de collimation (A), et un élément réfringent (3) étant disposé dans le trajet des rayons (SG) conduisant de l'oeil à l'optotype,
**caractérisé en ce que**
l'appareil de test de vision comprend plusieurs optotypes (S) à interposer à volonté dans le trajet des rayons (SG), **en ce que** les optotypes (S) sont placés sur un support (5) et sont pénétrés de lumière par une source lumineuse (11), **en ce que** le support (5) pour les symboles de visée est disposé de façon à être orientable ou translatable, **en ce que** des optotypes (S) quelconques placés sur le support (5) peuvent être interposés dans le trajet des rayons (SG),
**en ce que** l'élément réfringent (3) est une lentille achromatique recouvrant le champ de visée des deux yeux et interposée dans le trajet des rayons, ou un miroir concave dans le foyer duquel se trouve l'optotype (S) se trouvant dans le trajet des rayons, **en ce que** dans le trajet des rayons entre la source lumineuse (11) et l'optotype (S) un filtre (8), en particulier un filtre gris, est interposable par pivotement, **en ce que** la source de lumière crue (17) est placée de manière fixe à côté de l'optotype (S) pénétré de lumière se trouvant dans le trajet des rayons et **en ce que** au dessus du support (5) un ou plusieurs diaphragmes (4) sont dotés de différentes ouvertures de diaphragme qui peuvent être glissées au choix dans le trajet des rayons (SG) entre l'oeil et l'optotype (S).

2. Appareil de test de vision selon la revendication 1, **caractérisé en ce que** la source de lumière crue (17) et l'optotype (S) sont éclairés par la même source lumineuse (11).

3. Appareil de test de vision selon la revendication 2, **caractérisé en ce que** la source de lumière crue (17) comprend un guide de lumière qui est couplé à la source lumineuse (11) via un miroir séparateur ou un cube séparateur (14).

4. Appareil de test de vision selon l'une quelconque des revendications 2 ou 3, **caractérisé en ce que** l'intensité lumineuse de la source de lumière crue (17) est réglable par un diaphragme (15) rétractable dans le trajet des rayons vers le guide de lumière.

5. Appareil de test de vision selon l'une quelconque des revendications 1 à 4, **caractérisé en ce que** la luminosité de la source lumineuse (11) est enregistrée par un appareil de mesure (10) et est ajustable en fonction d'une valeur de consigne prédéfinie.

6. Appareil de test de vision selon l'une quelconque des revendications 1 à 5, **caractérisé en ce que** un disque dépoli est interposé dans le trajet des rayons (SG) et sous l'optotype (S).

7. Appareil de test de vision selon l'une quelconque des revendications 1 à 6, **caractérisé en ce que** le support (5) des optotypes (S) est un disque sur le périmètre duquel sont répartis les optotypes.

8. Appareil de test de vision selon la revendication 7, **caractérisé en ce que** le disque (5) est composé d'un matériau transparent.

9. Appareil de test de vision selon l'une quelconque des revendications 1 à 8, **caractérisé en ce que** le diaphragme (4) est sous la forme d'un disque ajouré.

10. Appareil de test de vision selon les revendications 7 et 9 ou les revendications 8 et 9, **caractérisé en ce que** le disque de diaphragme (4) et le disque du support de test de vision (5) sont disposés parallèlement à l'axe et soit de manière coaxiale soit à distance l'un de l'autre.

11. Appareil de test de vision selon l'une quelconque des revendications 7 à 10, **caractérisé en ce que** le filtre (8) repose sur un autre disque (7) qui est placé de façon coaxiale par rapport au disque de diaphragme (4) ou au disque du support d'optotype (5).

12. Appareil de test de vision selon l'une quelconque des revendications 7 à 11, **caractérisé en ce que** tous les disques (4, 5, 7) sont entraînés par moteur (M).

13. Appareil de test de vision selon l'une quelconque des revendications 7 à 12, **caractérisé en ce que** l'entraînement du disque ou les entraînements (M) des disques sont commandés par ordinateur.

14. Appareil de test de vision selon l'une quelconque des revendications 1 à 13, **caractérisé en ce que**, au dessus des optotypes, sont prévues une ou plusieurs sources lumineuses (19) qui forment des points de fixation à côté de l'optotype (S) au moyen de systèmes de reproduction .

15. Appareil de test de vision selon la revendication 14, **caractérisé en ce que** les sources lumineuses (19) sont des diodes.

16. Appareil de test de vision selon l'une quelconque des revendications 1 à 15, **caractérisé en ce que** dans le trajet des rayons (SG) un ou plusieurs diaphragmes (21) sont rétractables entre l'oeil et l'optotype (S) à des fins de recouvrement d'un oeil.

17. Appareil de test de vision selon l'une quelconque des revendications 1 à 16, **caractérisé en ce que** des couples de lentilles ménisques (20) sont disposés de manière à pouvoir interposés par pivotement dans le trajet des rayons (SG) devant les yeux.
